# EUROPEAN PATENT APPLICATION

(11) **EP 4 122 501 A2**
(43) Date of publication of application: **25.01.2023**
(21) Application number: 22186341.8
(22) Date of filing: 21.07.2022
(51) Int. Cl.: A61L 2/08, A61L 2/10, A61L 2/24

(54) **SAFE STERILIZATION UNIT AND DOUBLE-SURFACE STERILIZATION DEVICE INCLUDING SAME**

(30) Priority: 23.07.2021 TW 110127128; 04.07.2022 TW 111207087 U
(71) Applicant: Swanson Technologies Corporation, Taipei City 114 (TW)
(72) Inventor: Wang, Hsuan-Hui, 114 Taipei City (TW); Chang, Shih-Yi, 114 Taipei City (TW); Liao, Jung-Chen, 114 Taipei City (TW); Wu, Tung-Yang, 114 Taipei City (TW)
(74) Representative: 2K Patentanwälte Blasberg Kewitz & Reichel

(57) **Abstract**

The present invention provides a safe sterilization unit, including a bearing portion, a light source portion and at least one safety switch. The bearing portion includes two surfaces corresponding to each other; the light source portion is configured to generate sterilization light, and is disposed on one surface of the bearing portion; and the safety switch is disposed on one side surface, provided with the light source portion, of the bearing portion, and is connected with the light source portion. The safety switch is disposed to sense light to generate a sensing message, and to regulate power supply of the light source portion according to the sensing message.

## Description

### [FIELD OF TECHNOLOGY]

The present invention relates to a safe sterilization unit and a double-surface sterilization device including the same, in particular to a safe sterilization unit disposed to regulate power supply of a light source portion by a safety switch, and a double-surface sterilization device including the safe sterilization unit.

### [PRIOR ART]

Ultraviolet rays, belonging to a type of invisible light, cannot be detected by human eyes due to shorter wavelength than blue rays and purple rays. Based on basic physical properties of light, the shorter the wavelength of the light is, the higher the relative frequency of the light is, which also means higher energy. For an example of blue rays belonging to light with higher energy in visible light, the public generally worry about damage to eyes due to energy in the blue rays. While ultraviolet rays have much shorter wavelength and much higher energy than blue rays. Deoxyribonucleic acids (DNAs) of microorganisms can be destroyed with sufficient high energy in ultraviolet rays, causing that the DNAs are broken and cannot be replicated and the microorganisms lose physiological functions and die. As a result, the ultraviolet rays are often used for disinfection and sterilization.

In view of the function of ultraviolet rays in disinfection and sterilization, in recent years, ultraviolet-C light-emitting diode (UVC LED) lamps featuring small size, quick start, and moderate power are developed to replace traditional mercury lamps.

### [SUMMARY]

In accordance with the content in the prior art, the inventor further finds that the chance of contacting ultraviolet rays during use is increased due to the improvement in convenience of an ultraviolet lamp in irradiation; and if the lamp is accidentally turned on to emit ultraviolet rays when disinfection is not needed, high energy of the ultraviolet rays will also cause harm to a human body. Therefore, how to prevent a user from being irradiated by ultraviolet rays while disinfecting and sterilizing objects with the ultraviolet rays has become an urgent issue to be solved in the technical field.

In view of this, one aspect of the present invention provides a safe sterilization unit, including: a bearing portion comprising two surfaces corresponding to each other; a light source portion configured to generate sterilization light, and disposed on one surface of the bearing portion; and at least one safety switch disposed on one side surface, provided with the light source portion, of the bearing portion, and connected with the light source portion, where the safety switch is disposed to sense light to generate a sensing message, and to regulate power supply of the light source portion according to the sensing message.

According to an embodiment of the present invention, the safe sterilization unit further includes a lens portion disposed to contact the bearing portion and cover the light source portion, and made from a vinyl aromatic and conjugated diene cyclic block copolymer.

According to an embodiment of the present invention, the safe sterilization unit further includes a shell formed with an opening communicating with the outside, where the bearing portion is disposed in the shell, and the light source portion is disposed on one surface, facing the opening, of the bearing portion.

According to an embodiment of the present invention, the safety switch includes an infrared emitting diode and an infrared receiving diode, where the sterilization light and infrared rays emitted by the infrared emitting diode both pass through the opening, and the safety switch generates the sensing message according to an infrared ray receiving condition of the infrared receiving diode.

According to an embodiment of the present invention, the safe sterilization unit further includes a light-transmitting portion disposed at the opening.

According to an embodiment of the present invention, the shell includes a lens portion disposed on the opening, and covering the light source portion and the safety switch; and a lens is a Fresnel lens.

According to an embodiment of the present invention, the lens portion is disposed on a lamp bead of the light source portion.

According to an embodiment of the present invention, the safe sterilization unit further includes a moving unit connected with the light source portion to move an irradiation block of the light source portion.

According to an embodiment of the present invention, the light source portion includes a light-emitting lamp body for emitting ultraviolet rays.

Another aspect of the present invention provides a double-surface sterilization device, including a sterilization device body, and two safe sterilization units as described above. The sterilization device body includes an outer shell, and a power supply portion disposed inside the outer shell to regulate input power supply. The safe sterilization units are electrically connected to the power supply portion to obtain a driving power supply, and are respectively disposed on two opposite sides of the outer shell to form a first sterilization zone on a first side of the outer shell and a second sterilization zone on a second side of the outer shell.

According to an embodiment of the present invention, a position, corresponding to the second disinfection zone, of the first side of the sterilization device body is provided with a first support frame; and
a position, corresponding to the second disinfection zone, of the second side of the sterilization device body is provided with a second support frame.

According to an embodiment of the present invention, the first support frame is pivoted on the first side to adjust an opening and closing angle of the first support frame relative to a surface of the first side, and the second support frame is pivoted on the second side to adjust an opening and closing angle of the second support frame relative to a surface of the second side.

According to an embodiment of the present invention, the outer shell has the first side formed with a first accommodating groove for accommodating the first support frame, and the second side formed with a second accommodating groove for accommodating the second support frame.

According to an embodiment of the present invention, the sterilization device body includes two sliding mechanisms respectively disposed on the first side and the second side; and each of the two sliding mechanisms includes sliding rails disposed on the sterilization device body, and a sliding block disposed on the sliding rails for bearing the corresponding safe sterilization unit.

According to an embodiment of the present invention, the sterilization device body includes two guide grooves respectively formed in the first side and the second side, respectively allowing shells of the safe sterilization units on two sides to pass through, and limiting the movement stroke of the safe sterilization units.

According to an embodiment of the present invention, the sliding rails are formed on two sides of each of the guide grooves such that the outer shell protrudes toward an inner space, two sides of each of the sliding blocks are formed with grooves for the sliding rails to be clamped, and the sliding block is limited to move linearly by the sliding rails.

According to an embodiment of the present invention, the sterilization device body includes two positioning mechanisms respectively disposed on the first side and the second side for fixing an object to be sterilized.

According to an embodiment of the present invention, each of the positioning mechanisms includes a positioning column inserted into the outer shell, and a positioning elastic piece with one end fixed to the positioning column; and the positioning elastic piece is opened relative to the other end of the positioning column and elastically returns against a surface of the outer shell to provide clamping.

According to an embodiment of the present invention, the power supply portion is connected to an external power supply via an electrical connector disposed on the outer shell.

According to an embodiment of the present invention, an inner side of the outer shell is provided with an accommodating mechanism, the accommodating mechanism is open toward a bottom side of the outer shell, and a draw-out hook is disposed in the accommodating mechanism.

The safe sterilization unit in the present invention enables the light source portion for emitting the ultraviolet rays to fully exert its sterilization function without causing accidental damage to a human body through a mechanism that the infrared emitting diode and the infrared receiving diode act as the safety switch.

### [BRIEF DESCRIPTION OF DRAWINGS]

Various features and components in the drawings are not drawn to scale, and are only drawn to best present specific features and components related to the present invention. In addition, the same or similar components and parts are referred to by reference numbers of the same or similar components in different drawings.
FIG. 1 is a schematic cross-sectional view of a first embodiment of a safe sterilization unit in the present invention.
FIG. 2 is a schematic diagram of a control circuit of a safety switch and a light source portion in the first embodiment of the safe sterilization unit in the present invention.
FIGS. 3A to 3B are schematic diagrams of the first embodiment of the safe sterilization unit during use in the present invention.
FIG. 4 is a schematic cross-sectional view of an embodiment 1-1 of the safe sterilization unit in the present invention.
FIG. 5 is a schematic cross-sectional view of an embodiment 1-2 of the safe sterilization unit in the present invention.
FIG. 6 is a schematic cross-sectional view of an embodiment 1-3 of the safe sterilization unit in the present invention.
FIG. 7 is a schematic cross-sectional view of a second embodiment of the safe sterilization unit in the present invention.
FIGS. 8A to 8B are schematic cross-sectional views of a lens portion in the second embodiment of the safe sterilization unit in the present invention.
FIG. 9 is a schematic diagram of an appearance of an embodiment of a double-surface sterilization device in the present invention.
FIG. 10 is a schematic exploded view (1) of a structure of an embodiment of the double-surface sterilization device in the present invention.
FIG. 11 is a schematic exploded view (2) of a structure of an embodiment of the double-surface sterilization device in the present invention.
FIG. 12 is a schematic block diagram of an embodiment of a power supply portion in the present invention.

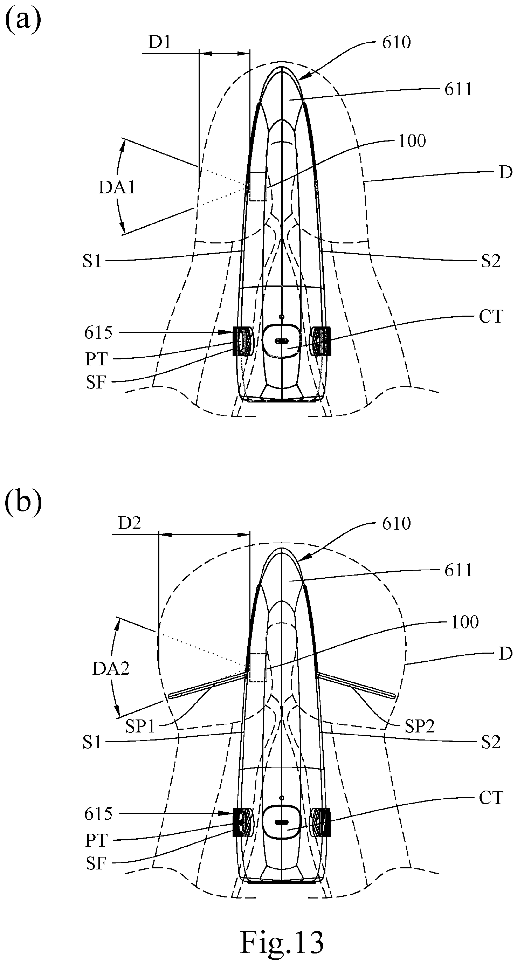

FIGS. 13A to 13B are schematic diagrams (1) of an embodiment of the double-surface sterilization device in a usage status according to the present invention.

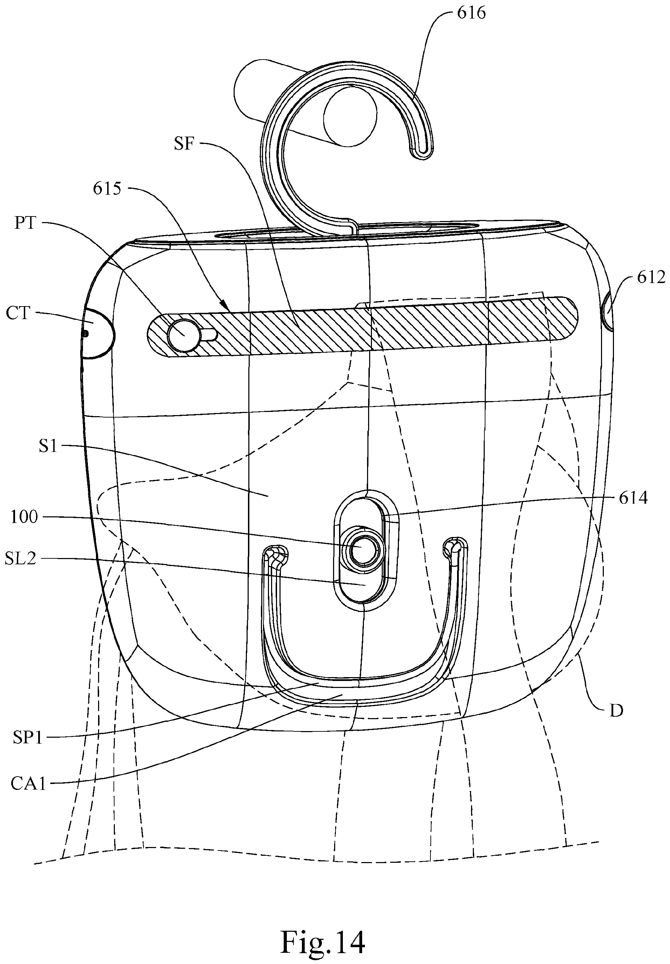

FIG. 14 is a schematic diagram (2) of an embodiment of the double-surface sterilization device in the usage status according to the present invention.
FIGS. 15A to 15B are schematic cross-sectional views of an embodiment of the double-surface sterilization device during use in the present invention.
FIG. 16 is a schematic diagram of an embodiment of the double-surface sterilization device in the usage status according to the present invention.

### [DETAILED DESCRIPTION OF THE EMBODIMENTS]

In order to make the technical connotation of the present invention more detailed and complete, the following describes the implementation patterns and specific embodiments of the present invention, but the description below is not the only form of implementing or using the specific embodiments of the present invention. Those of ordinary skill in the art can easily understand the necessary technical content of the present invention through the description below, and can change and modify the present invention in various ways to adapt to different uses and conditions without violating the spirit and scope of the present invention. Thus, the implementation patterns also belong to the claims of the present invention.

In the description of the present invention, unless the context dictates otherwise, "1" and "the" can also be construed as plural. In addition, within the scope of this description and the appended claims, unless otherwise stated, "disposed on an object" may be regarded as directly or indirectly contacting the surface of an object by attachment or in other forms, and the definition of the surface should be judged according to the context/paragraph semantics of the description and common knowledge in the field to which this description belongs.

In the description of the present invention, unless the context dictates otherwise, "contain", "include/comprise", "has/have" or "involve" is inclusive or open, and do not exclude other undescribed elements or method steps.

In the description of the present invention, the terms "middle", "upper", "lower", "front", "back", "left", "right", "vertical", "horizontal", "top", "bottom" , "inside", "outside", etc. for indicating orientations or positional relationships are based on orientations or positional relationships shown in the drawings, which are only for the convenience of describing the present invention and simplifying the description, rather than indicating or implying that the referred device or component must have a specific orientation and be constructed and operated in a specific orientation, and thus should not be construed as limiting the present invention.

In the description of the present invention, "disposed", "installed", "connected", "connection", "fixed", etc. should be understood in a broad sense, which may be, for example, fixed connection, detachable connection, mechanical connection, directly connected, indirectly connected via a middle medium, etc. Those of ordinary skill in the art can understand the specific meanings of the above terms in the present invention according to specific circumstances.

The programs mentioned in the present invention can all be executed by corresponding controllers (such as but not limited to charge and discharge control, logic control, or power control (such as pulse width modulation (PWM)), analog-to-digital and digital-to-analog conversion, etc.). The "controllers" may be processors or processors connected to storage units and executing corresponding programs, and control the operation of all components or devices according to software, firmware, or hardware configuration. Such controllers can be understood to be included in corresponding devices (such as a light source portion, a safety switch, a power supply portion, etc.) even if they are not mentioned in the description. Such controllers may be, for example, central processing units (CPUs), other programmable general-purpose or special-purpose microprocessors, digital signal processors (DSPs), programmable controllers, application specific integrated circuits (ASICs), other similar devices or combinations of these devices, which are not limited in the present invention.

### First Embodiment

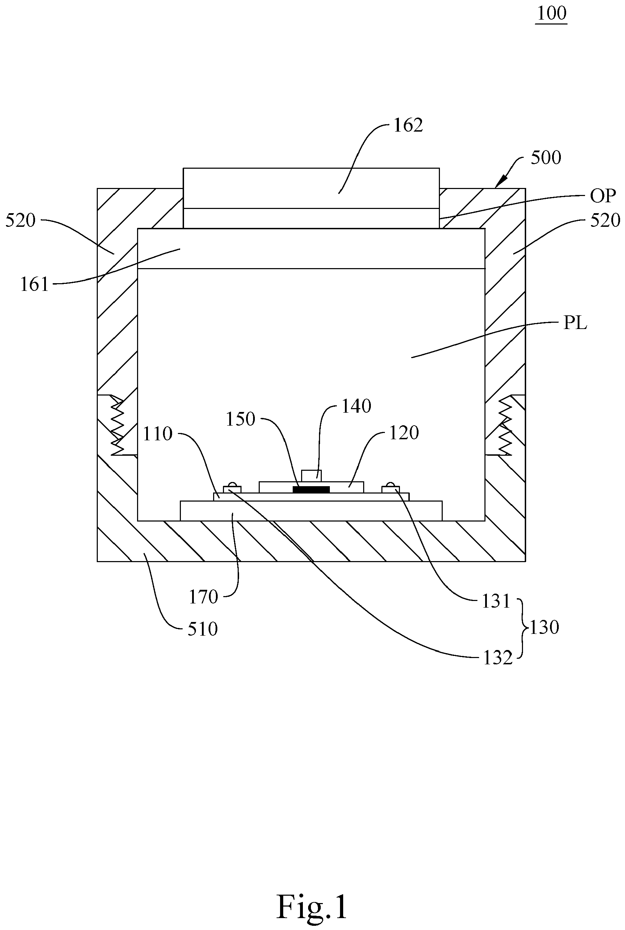

FIG. 1 shows a schematic cross-sectional view of a first embodiment of the present invention. A safe sterilization unit 100 includes a shell 500 , a bearing portion 110, at least one safety switch 130, a light source portion 140, and a moving unit 150.

The shell 500 is formed with an opening OP communicating with the outside, and has an accommodating space PL therein for accommodating the bearing portion 110, the safety switch 130, the light source portion 140, and the moving unit 150. In an embodiment, the shell 500 includes a bottom plate 170 disposed in the accommodating space, such that the bearing portion 110 is disposed on the bottom plate 170. Lead wires and related components including but not limited to driving chips or signal processing components can be disposed on or inside the bottom plate 170 according to requirements, and are electrically connected with the safety switch 130 and the light source portion 140; and specific use patterns of the components are not limited by the present invention. In an embodiment, the opening OP in the shell 500 is further provided with a light-transmitting portion 161 for light to pass through. The light-transmitting portion 161 is located on a light output path of the light source portion 140, and light emitted by an infrared emitting diode 131 of the safety switch 130, light received by an infrared receiving diode 132 of the safety switch, and light emitted by the light source portion 140 can all enter and exit from the shell 500 via a window portion, such that irradiation ranges of the safety switch 130 and the light source portion 140 are overlapped. In an embodiment, a material of the light-transmitting portion 161 may be quartz, a cyclic block copolymer (CBC), or other materials with equivalent application. In an embodiment, the safe sterilization unit 100 may or may not be provided with a lens portion 162 corresponding to the light source portion 140, which is not limited in the present invention.

In an embodiment, the shell 500 includes a base 510, and an extension portion 520 disposed on the base 510; and the extension portion 520 is reserved with the above-mentioned opening OP communicating with the outside.

The bearing portion 110 is disposed in the shell 500. The bearing portion 110 has upper and lower surfaces corresponding to each other, preferably disposed opposite to each other and preferably being parallel to each other; and the bearing portion 110 is of a plate structure, which is not limited herein. The light source portion 140 is disposed above a surface of the bearing portion 110, and is preferably a light-emitting lamp body. In this embodiment, the light source portion is the light-emitting lamp body for emitting ultraviolet rays. In detail, the bearing portion 110 is preferably a plate body with a heat dissipation function; a material of the bearing portion may be a heat-dissipating material well known in the art, such as a metal or alloy containing copper and/or aluminum, or a ceramic; and more preferably, the bearing portion is a heat-dissipating aluminum plate.

In an embodiment, a base plate 120 is further disposed between the bearing portion 110 and the light source portion 140; the base plate 120 may be made of the same or different material as or from the bearing portion 110, such as a metal or alloy containing copper and/or aluminum, or a ceramic; and in this embodiment, the base plate is made of the aluminum.

The safety switch 130 is disposed on one side surface, provided with the light source portion, of the bearing portion 110, and includes the infrared emitting diode 131, the infrared receiving diode 132 and a control integrated circuit (IC), where the infrared emitting diode 131 and the infrared receiving diode 132 are connected with the light source portion 140 via the control IC. The infrared emitting diode 131 and the infrared receiving diode 132 are disposed on an upper surface of the bearing portion 110.

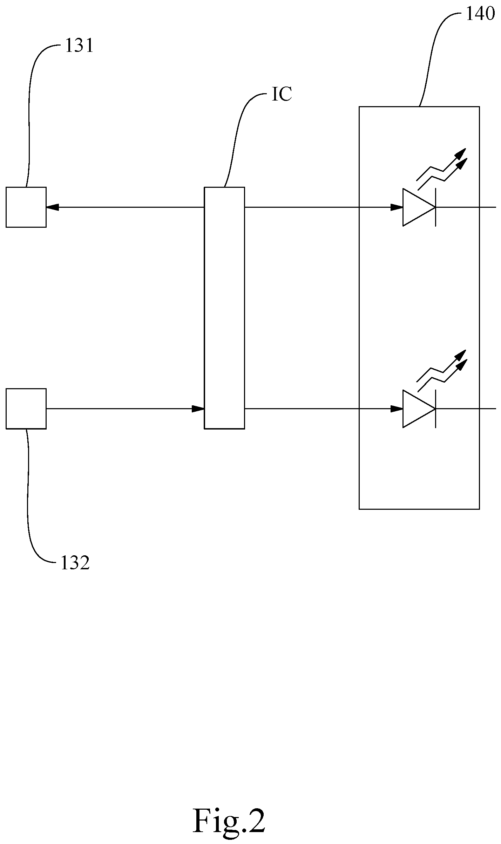

Due to emission wavelengths which are far apart, the ultraviolet rays and infrared rays will not interfere with each other in function. For a situation of use in conjunction with the safety switch 130, as shown in FIG. 2, the safety switch 130, according to a control signal provided by the control IC, outputs the infrared rays by the infrared emitting diode 131, and senses the infrared rays received by the infrared receiving diode and generates a sensing message by the infrared receiving diode 132, and then regulates power supply of the light source portion 140 by using the control IC according to the sensing message. In an embodiment, the regulation of power supply may include, for example, regulating power supply to driving power that cannot be felt by human eyes, regulating power supply to driving power, close to a starting voltage, of the light source portion, or turning off power supply of the light source portion. In this embodiment, the infrared emitting diode 131 and the infrared receiving diode 132 are respectively located on two sides of the light source portion 140, where the numbers or arrangement positions of infrared emitting diodes 131, infrared receiving diodes 132, and control ICs can be increased or moved according to requirements, which are not limited in the present invention.

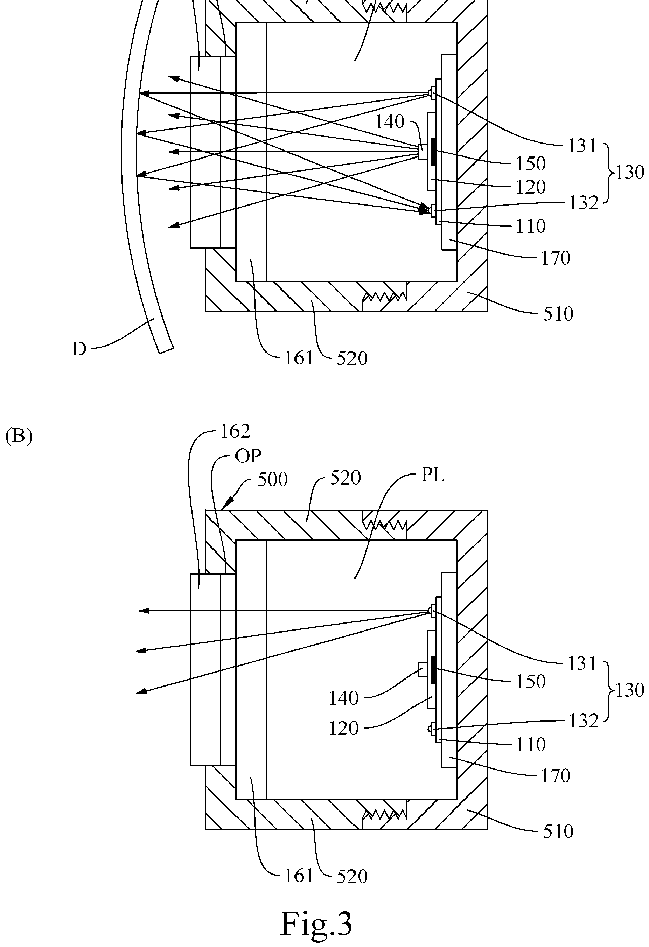

In an embodiment, the infrared emitting diode 131 and the infrared receiving diode 132 are respectively located on two sides of the light source portion 140. As shown in FIG. 3A, the safety switch 130 outputs infrared rays by the infrared emitting diode 131 through the light-transmitting portion. In the presence of an object D to be sterilized, the infrared receiving diode 132 will receive infrared rays reflected by the object D to be sterilized and generate a corresponding sensing message, and the control IC turns on the light source portion 140 after receiving the sensing message. While in FIG. 3B, in the absence of the object D to be sterilized or when the disinfected object D is removed, the infrared receiving diode 132 will not receive the infrared rays reflected by the object D to be sterilized, and the control IC does not turn on the light source portion 140 or reduces the power of the light source portion 140 when the sensing message is not received or the sensing message is lower than a set threshold, so as to avoid user's eye or skin damage due to accidental close contact.

In a case where a plurality of safety switches 130 are used, the conditions for turning on the light source portion 140 can be improved. When infrared emitting diodes 131 on two sides of the light source portion 140 are shielded at the same time, the light source portion 140 will be turned on and emits the ultraviolet rays for sterilization, otherwise, the light source portion 140 will not be turned on, so as to prevent children from accidentally touching the safety switches 130.

Furthermore, since light paths of the ultraviolet rays and the infrared rays are almost overlapped, as long as an area detected by the infrared emitting diode 131 of the safety switch 130 is almost the same as an ultraviolet irradiation range of the light source portion 140, a dead angle of detection can be avoided, and a situation that the object is irradiated in front of the light source portion 140, but is not detected by the safety switch 130 is prevented.

Then, the moving unit 150 in the safe sterilization unit 100 is connected with the light source portion 140, and preferably can be hidden in the base plate 120. The moving unit 150 can move or vibrate with the light source portion 140 in a small range, so as to move an irradiation block of the light source portion 140. Besides, a power source of the moving unit 150 can be provided by a motor, which is not limited in the present invention.

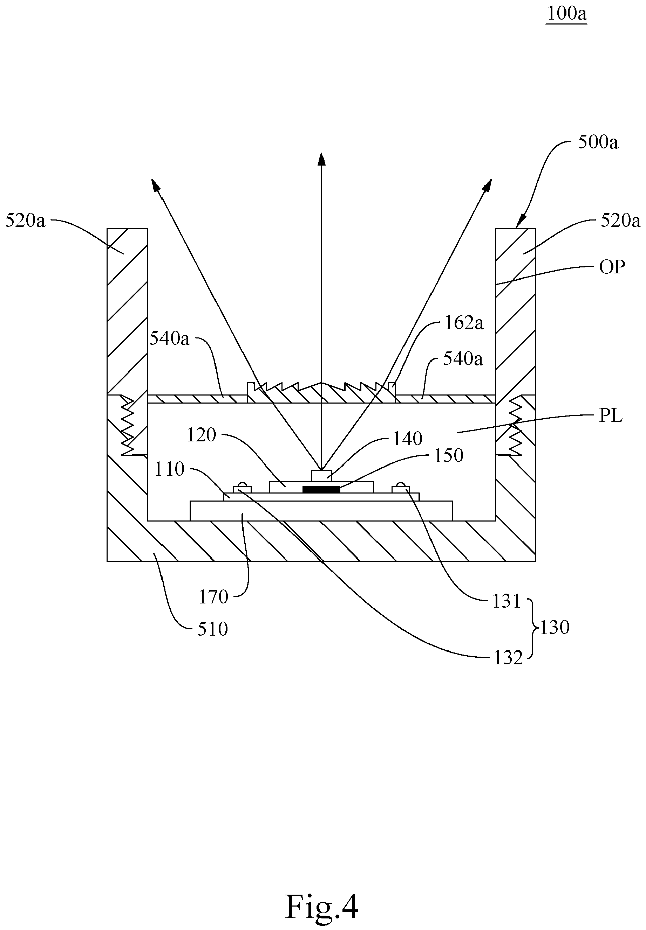

FIG. 4 shows a schematic cross-sectional view of an embodiment 1-1 of the present invention. Main components of a safe sterilization unit 100a in the embodiment 1-1 are the same as those in the first embodiment and have the same functions; and differences are that a support frame 540a is disposed inside an extension portion 520a of a shell 500a for erecting a lens portion 162a as a window portion through which light passes, and the lens portion 162a is located on a light output path of the light source portion 140. Preferably, the extension portion 520a is not fixedly connected with a base 510a; and more preferably, the extension portion 520a can move back and forth along the base 510a, thereby adjusting a distance between the lens portion 162a and the light source portion 140.

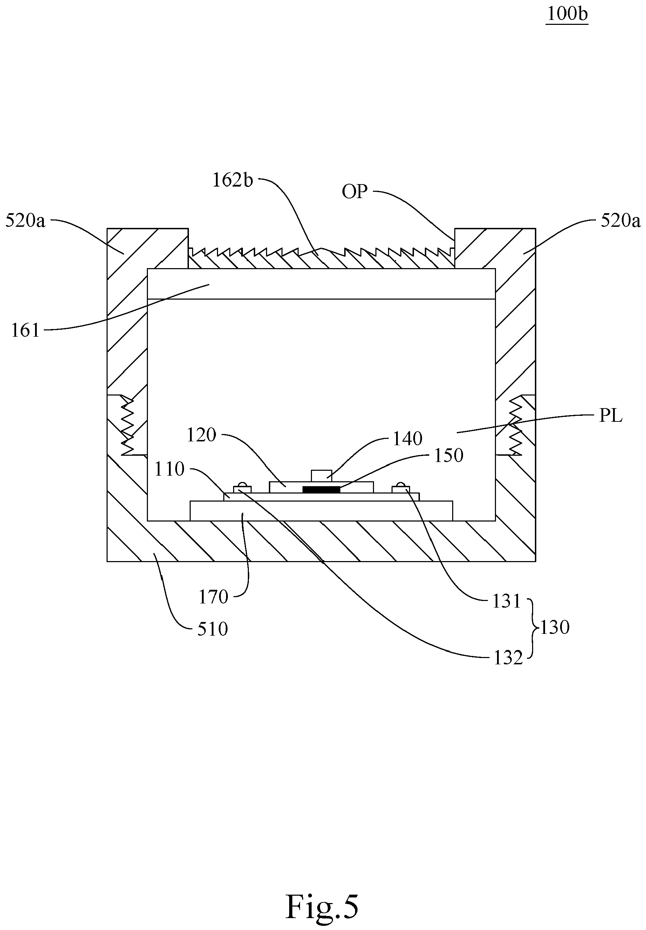

FIG. 5 shows a schematic cross-sectional view of an embodiment 1-2 of the present invention. A difference between this embodiment and the previous embodiment is that a lens portion 162b in this embodiment is directly disposed on the light-transmitting portion 161 of the extension portion 520; and in another embodiment, the lens portion 162b can be integrally formed and combined on the light-transmitting portion 161, which is not limited in the present invention. For the embodiment in FIG. 5, the lens portion 162b and the light source portion 140 are arranged at an interval.

In another aspect, the lens portion 162, 162a or 162b is preferably a Fresnel lens; and as used herein, the "Fresnel lens" is a variant lens formed by dividing numerous concentric circle lines (Fresnel zones) on a lens, can be divided into types of point focusing and line focusing, and can be divided into curved and plano Fresnel lenses according to geometric patterns of lenses. The Fresnel lens can achieve short-length focusing or collimation under the condition of reducing its material.

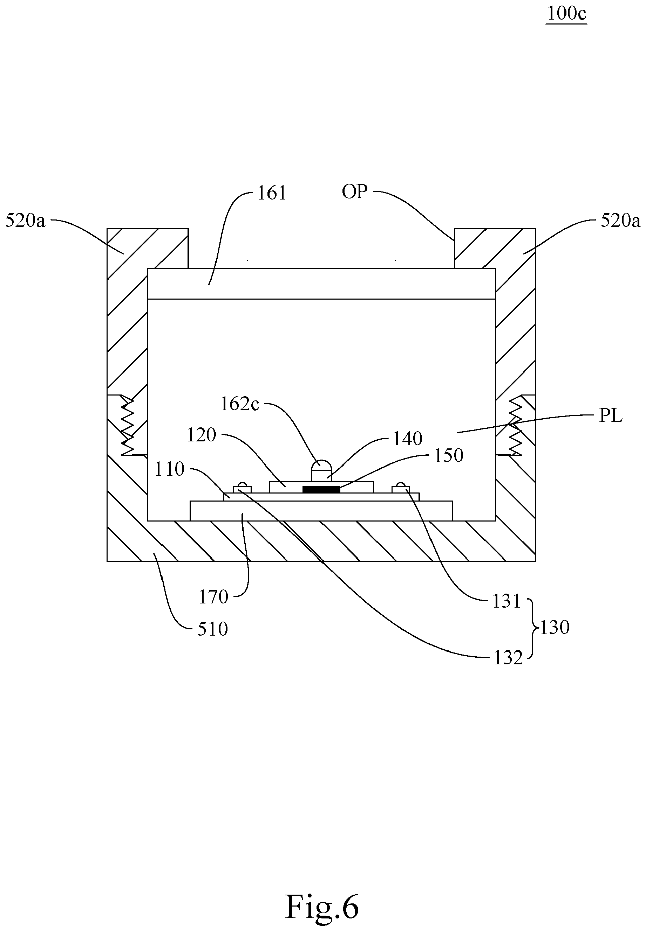

FIG. 6 shows a schematic cross-sectional view of an embodiment 1-3 of the present invention. A difference between this embodiment and the previous embodiment is that a lens portion 162c in this embodiment is directly disposed on the lamp bead of the light source portion 140; and in another embodiment, the lens portion 162b can be integrally formed and combined on the lamp bead of the light source portion 140 in a manufacture procedure directly, which is not limited in the present invention.

A material of the lens portion 162, 162a, 162b or 162c is a polymer, preferably a copolymer, and more preferably a vinyl aromatic (such as styrene)-conjugated diene (such as butadiene) cyclic block copolymer. The "block copolymer" belongs to a sub-category of copolymers, and includes two or more chain segments formed by single structural units. Besides, the vinyl aromatic-conjugated diene cyclic block copolymer may be prepared by an anionic polymerization technology or a catalytic hydrogenation technology, which is not limited in the present invention.

### Second Embodiment

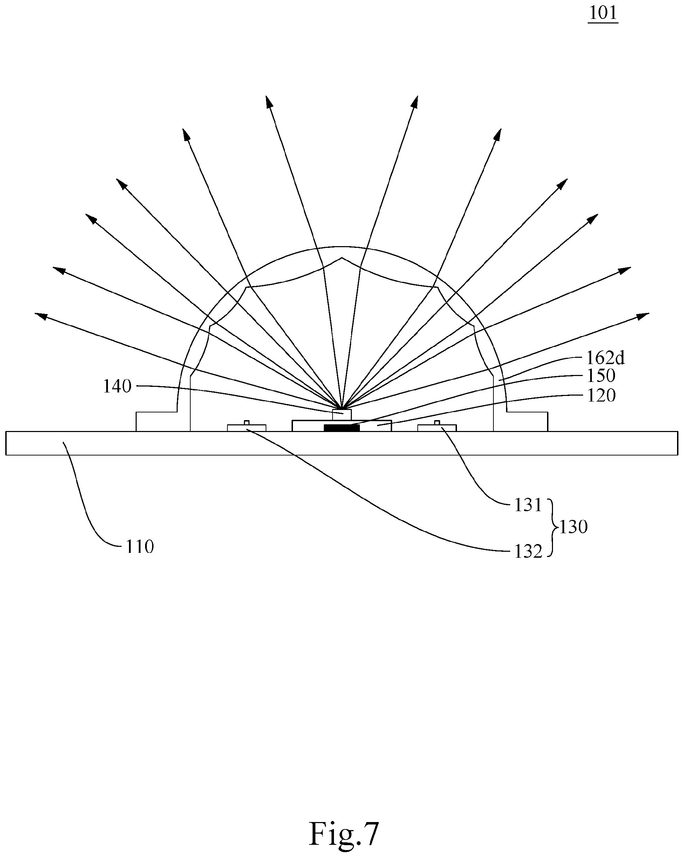

FIG. 7 shows a schematic cross-sectional view of a second embodiment of the present invention. Main components of a safe sterilization unit 101 in the second embodiment are similar to main components of the safe sterilization unit 100 in the first embodiment and have similar functions. Differences between the first embodiment and the second embodiment are that the bearing portion 110 is not disposed in the shell, and a lens portion 162d is disposed in contact with an upper surface of the bearing portion 110 and covers the light source portion 140; the so-called "cover", as used herein, refers generally to a relative relationship that an object at least partially covers a surface area of another object; and it may be a relative relationship without direct contact, point contact or surface contact. For this embodiment, the lens portion 162d and the light source portion 140 are arranged at an interval.

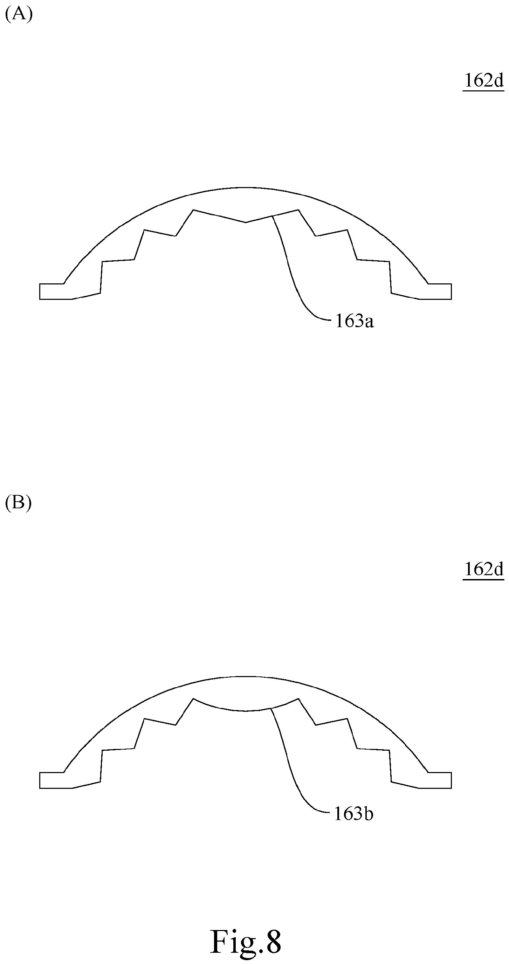

In another aspect, the lens portion 162d is a Fresnel lens; and as used herein, the "Fresnel lens" is a variant lens formed by dividing numerous concentric circle lines (Fresnel zones) on a lens, can be divided into types of point focusing and line focusing, and can be divided into curved and plano Fresnel lenses according to geometric patterns of lenses. The Fresnel lens can achieve short-length focusing or collimation under the condition of reducing its material. FIGS. 8A to 8B are cross-sectional views of a lens portion in an embodiment of the present invention. Referring to FIGS. 8A to 8B, the lens portion 162d is a curved Fresnel lens, and has a lower surface engraved with a plurality of tooth structures (the sizes, shapes, angles and scales of most tooth structures shown in the figures are all used for illustration only, and do not present actual geometric parameters); and however, according to different patterns, part of the tooth structures of the curved Fresnel lens may be conical structures 163a (as shown in FIG. 8A), or arc structures 163b (as shown in FIG. 8B).

A material of the lens portion 162d is a polymer, preferably a copolymer, and more preferably a vinyl aromatic (such as styrene)-conjugated diene (such as butadiene) cyclic block copolymer. As used herein, the "block copolymer" belongs to a sub-category of copolymers, and includes two or more chain segments formed by single structural units. Besides, the vinyl aromatic-conjugated diene cyclic block copolymer may be prepared by an anionic polymerization technology or a catalytic hydrogenation technology, which is not limited in the present invention.

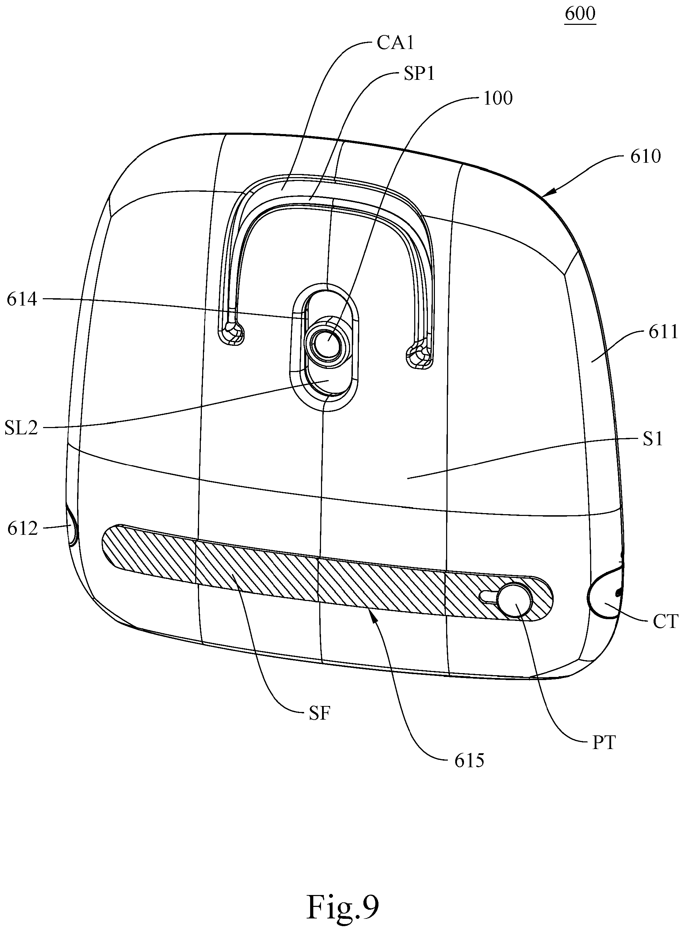

The safe sterilization unit in the present invention has been described in detail above, and the following will describe a double-surface sterilization device corresponding to the present invention in detail with specific embodiments. FIG. 9, FIG. 10 and FIG. 11 respectively show a schematic diagram of an appearance, a schematic exploded view (1) of a structure, and a schematic exploded view (2) of a structure of a double-surface sterilization device 600 using the safe sterilization unit 100 in the first embodiment of the present invention. Referring to FIGS. 1 to 6 and FIGS. 9 to 11, the double-surface sterilization device 600 disclosed in this embodiment mainly includes a sterilization device body 610, and two safe sterilization units 100, as described above, respectively disposed on two sides of the sterilization device body 610. In this embodiment, although the number of safe sterilization units 100 is set to be two, more than two safe sterilization units 100 also can be disposed according to actual needs in the present invention to increase the sterilization range, or the number of sterilization areas is increased to disinfect a plurality of materials or clothing materials to be sterilized at the same time. In addition, in a preferred implementation pattern, according to the safe sterilization units 100 on two sides of the double-surface sterilization device 600, only under the condition that infrared emitting diodes 131 on two sides are shielded at the same time, light source portions 140 on two sides will be turned on together and emit ultraviolet rays for sterilization, otherwise, the light source portions 140 on the two sides will not be turned on.

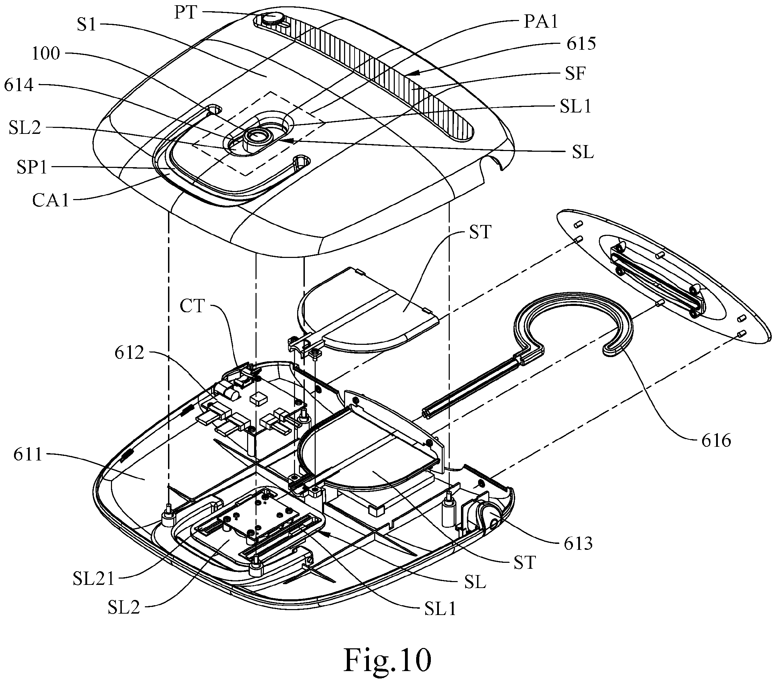

The sterilization device body 610 mainly includes an outer shell 611, a power supply portion 612 disposed inside the outer shell 611 for regulating input power supply, and an operation unit 613 connected to the power supply portion 612. The power supply portion 612 is used to provide a power supply required by each apparatus in the double-surface sterilization device 600 according to the embodiment. In an embodiment, the power supply portion 612 can be implemented in the form of a main control circuit board, and is configured to be connected with each apparatus of the double-surface sterilization device 600; a controller of each apparatus can be optionally configured on the main control circuit board (such as a processor, a logic controller, an analog-to-digital converter, a digital-to-analog converter, a switch controller, etc.); of course, such controllers can also be optionally configured on respective independent circuit boards; and such changes are all within the scope of protection of the present invention. In an embodiment, the power supply portion 612 is connected to an external power supply via an electrical connector CT disposed on the outer shell 611, where the external power supply may be, for example, a relay, any connecting wire, or a plug apparatus, which is not limited in the present invention. In yet another embodiment, as shown in FIG. 10, the power supply portion 612 includes a power supply module A1 connected to the electrical connector CT, a charging module A2 connected to the power supply module A1, and a battery A3 connected to the charging module A2. In an embodiment, the power supply module A1 may include, but is not limited to, for example, a rectifier, a transformer, an isolator, a protection circuit or other similar apparatuses; the charging module A2 may include, but is not limited to, for example, any constant current circuit, constant voltage circuit, protection circuit, temperature detection apparatus, or feedback circuit for detecting charging capacity; and the battery A3 may include, but is not limited to, for example, a nickel-cadmium battery, a nickel-metal hydride battery, a lithium-ion battery, a lithium-polymer battery, or a lithium iron phosphate battery, which are not limited in the present invention. The operation unit 613 may be, for example, a key switch, an operation panel, a touch panel, etc., and is used as a man-machine interface to input control commands (including turn-on, turn-off, power adjustment, etc.), which is not limited in the present invention.

The safe sterilization unit 100 is as described in the previous embodiment, and the same parts will not be repeated. The safe sterilization unit 100 is electrically connected to the power supply portion 612 to obtain a driving power supply. In an embodiment, the safe sterilization unit 100 can be directly electrically connected to the power supply portion 612 by hidden wire arrangement. In another embodiment, the safe sterilization unit 100 can obtain a driving power supply from the power supply portion 612 by wireless power supply (such as a coupling coil), which is not limited in the present invention. In order to achieve the function of double-surface sterilization, two safe sterilization units 100 are respectively disposed on two opposite sides of the outer shell 611 to form a first sterilization zone PA1 on a first side S1 of the outer shell 611 and a second sterilization zone PA2 on a second side S2 of the outer shell 611. The first side S1 and the second side S2 (including types, quantities, shapes, and configuration positions of apparatuses and mechanisms) of the safe sterilization unit 100 in this embodiment may be symmetrical or asymmetrical in design, which is not limited in the present invention. For the convenience of description, and to avoid excessive repetition of substantially same content, most of technical features in the following are described with one side or only one side is marked in the figure, but such configuration is not limited to bilateral symmetry, which is clearly indicated in advance here.

In an embodiment, in order to move the safe sterilization unit 100 to a preferred position in cooperation with corresponding materials or clothing materials to be sterilized, the sterilization device body 610 includes two sliding mechanisms SL respectively disposed on the first side S1 and the second side S2; and each of the sliding mechanisms SL includes sliding rails SL1 disposed on the sterilization device body 610, and a sliding block SL2 disposed on the sliding rails SL1 for bearing the corresponding safe sterilization unit 100. Specifically, the sterilization device body 610 includes two guide grooves 614 respectively formed in the first side S1 and the second side S2, and the two guide grooves 614 respectively allow openings of the safe sterilization units 100 on two sides to pass through and limit the movement stroke of the safe sterilization units 100; and the sliding rails SL1 are formed on two sides of each of the guide grooves 614 such that the outer shell 611 protrudes toward an inner space, two sides of the sliding block SL2 are provided with grooves SL21 for the sliding rails SL1 to be clamped, and the sliding block SL2 is limited to move linearly by the sliding rails SL1. Each of the grooves SL21 has two closed ends and a limited length, thereby limiting the relative movement stroke between the sliding rails SL1 and the sliding block SL2.

In an embodiment, in order to increase the sterilization range of the safe sterilization unit 100 on the first disinfection zone PA1 and the second disinfection zone PA2, a position, corresponding to the first disinfection zone PA1, of the first side S1 of the sterilization device body 610 is provided with a first support frame SP1, and/or a position, corresponding to the second disinfection zone PA2, of the second side S2 of the sterilization device body 610 is provided with a second support frame SP2, so as to increase a distance between the materials or clothing materials to be sterilized and the safe sterilization unit 100; and based on a characteristic that a beam angle of sterilization light is increased with the distance when exceeding 0°, the sterilization range is increased.

Further, in an embodiment, the first support frame SP1 is pivoted on the first side S1 to adjust an opening and closing angle of the first support frame SP1 relative to a surface of the first side S1, and the second support frame SP2 is pivoted on the second side S2 to adjust an opening and closing angle of the second support frame SP2 relative to a surface of the second side S2. In order to improve the appearance of a product, the outer shell 611 has the first side S1 formed with a first accommodating groove CA1 for accommodating the first support frame SP1, and the second side S2 formed with a second accommodating groove CA2 for accommodating the second support frame SP2.

In an embodiment, in order to fix the positions of the materials or clothing materials to be sterilized, the sterilization device body 610 includes two positioning mechanisms 615 respectively disposed on the first side S1 and the second side S2 for fixing the materials or clothing materials to be sterilized. In an embodiment, each of the positioning mechanisms 615 includes a positioning column PT inserted into the outer shell 611, and a positioning elastic piece SF with one end fixed to the positioning column PT; and the positioning elastic piece SF is opened relative to the other end of the positioning column PT and elastically returns against a surface of the outer shell 611 to provide clamping.

In an embodiment, in order to facilitate the double-surface sterilization device 600 in the present invention to achieve a hanging function, an inner side of the outer shell 611 is provided with an accommodating mechanism ST, the accommodating mechanism ST is open toward a bottom side of the outer shell 611, and a draw-out hook 616 is disposed in the accommodating mechanism ST, such that the hanging function is achieved by the draw-out hook 616.

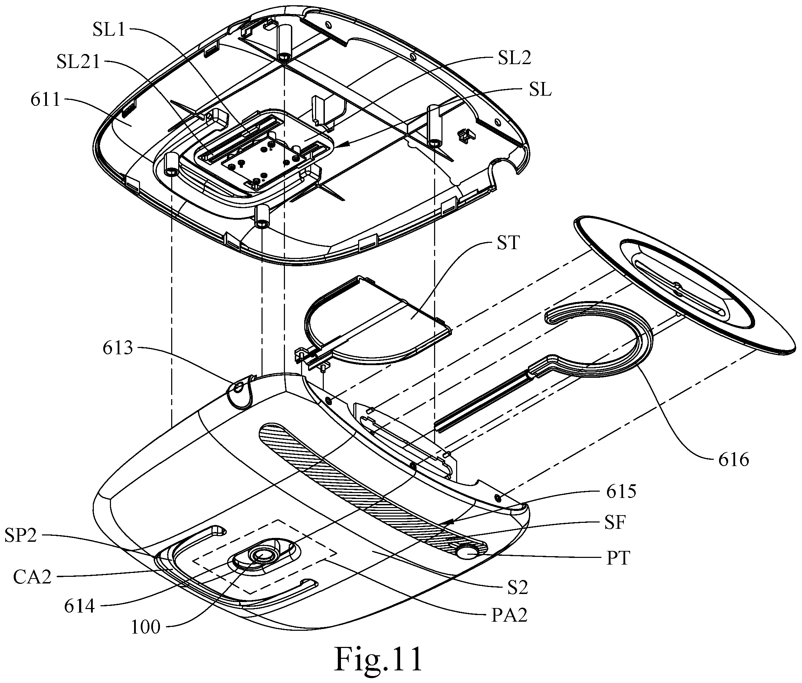

With respect to a usage mode of the double-surface sterilization device in the present invention, FIGS. 11A to 11B show schematic diagrams (1) of an embodiment of the double-surface sterilization device in a usage status according to the present invention. As shown in FIG. 9A, when the first support frame SP1 is closed, the first support frame SP1 is accommodated in the first accommodating groove CA1 of the first side S1, a distance D1 between the materials or clothing materials to be sterilized and the safe sterilization unit 100 on the first side S1 is relatively short, and a disinfection range DA1 according to a beam angle of sterilization light is as shown in the figure. As shown in FIG. 9B, when the first support frame SP1 is opened, there is an opening and closing angle α between the first support frame SP1 and the first side S1, a distance D2 between the materials or clothing materials to be sterilized and the safe sterilization unit 100 on the first side S1 is longer than the distance D1 because the materials or clothing materials to be sterilized are supported by the first support frame SP1, and a disinfection range DA2 is wider than the disinfection range DA1 under the premise of the same beam angle of the sterilization light, thereby increasing the sterilization range.

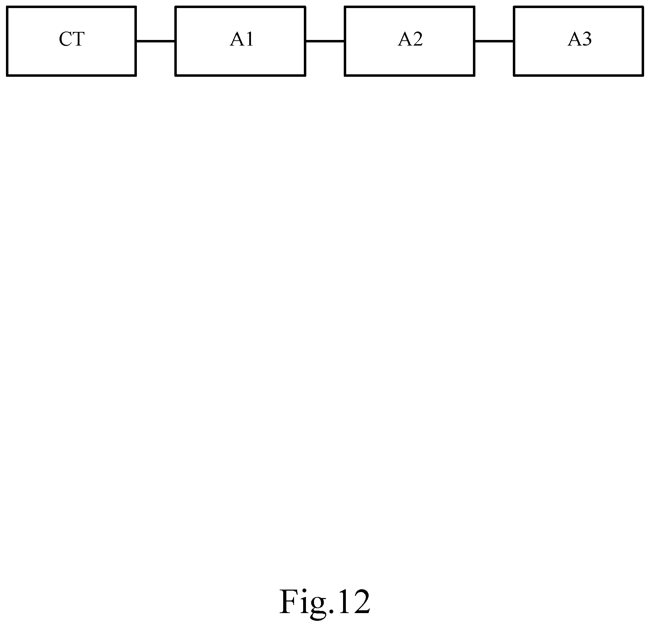

In addition to the above usage mode, the double-surface sterilization device 600 in the present invention can also be hung for use. FIG. 12 shows a schematic diagram (2) of an embodiment of the double-surface sterilization device in a usage status according to the present invention. In an embodiment, the double-surface sterilization device 600 in the present invention can draw out the draw-out hook 616 with an opening in a bottom side from the accommodating mechanism ST, and the double-surface sterilization device 600 is hung upside down on a bracket (such as a clothes hanger) by the draw-out hook 616. In order to fix the materials or clothing materials to be sterilized to the double-surface sterilization device 600 when the double-surface sterilization device 600 is hung upside down on the bracket, the sterilization device body 610 includes two positioning mechanisms 615 respectively disposed on the first side S1 and the second side S2 for fixing the materials or clothing materials to be sterilized, and a part of the materials or clothing materials to be sterilized are respectively clamped by the positioning mechanisms 615 on the two sides, such that the materials or clothing materials to be sterilized are fixed to the sterilization device body 610.

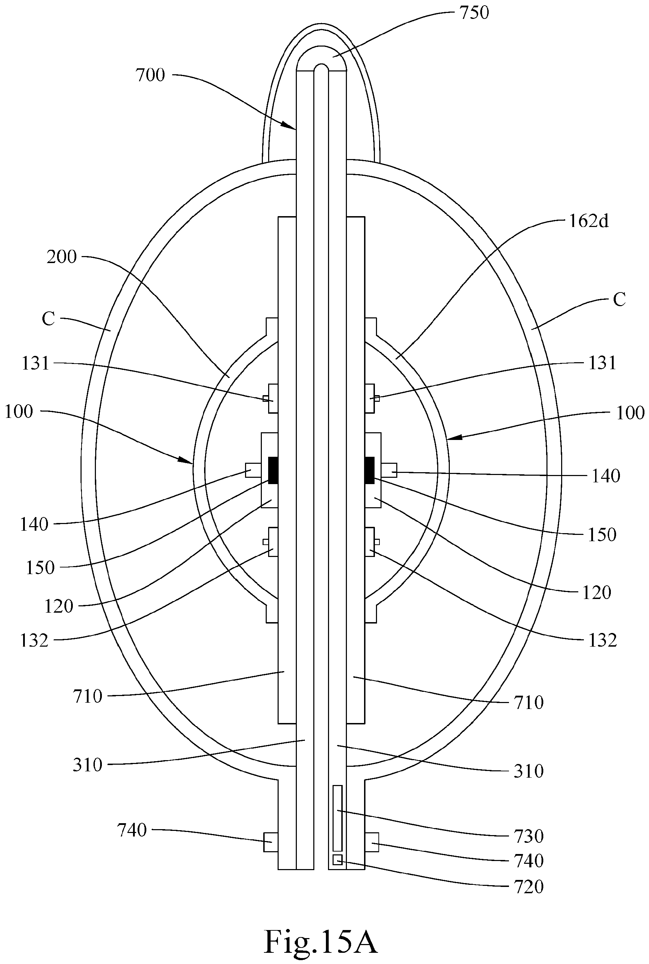

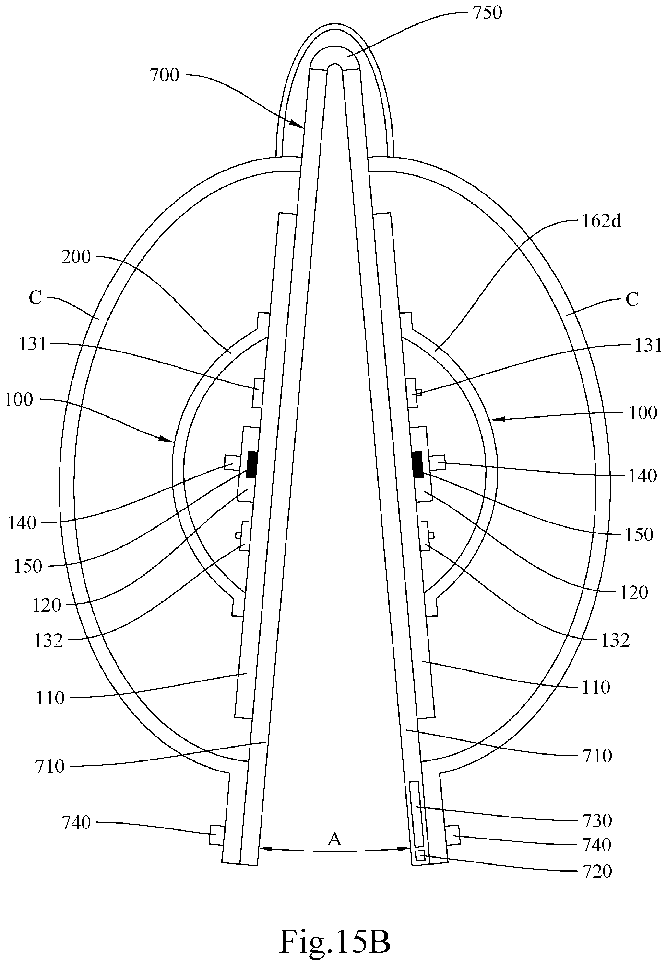

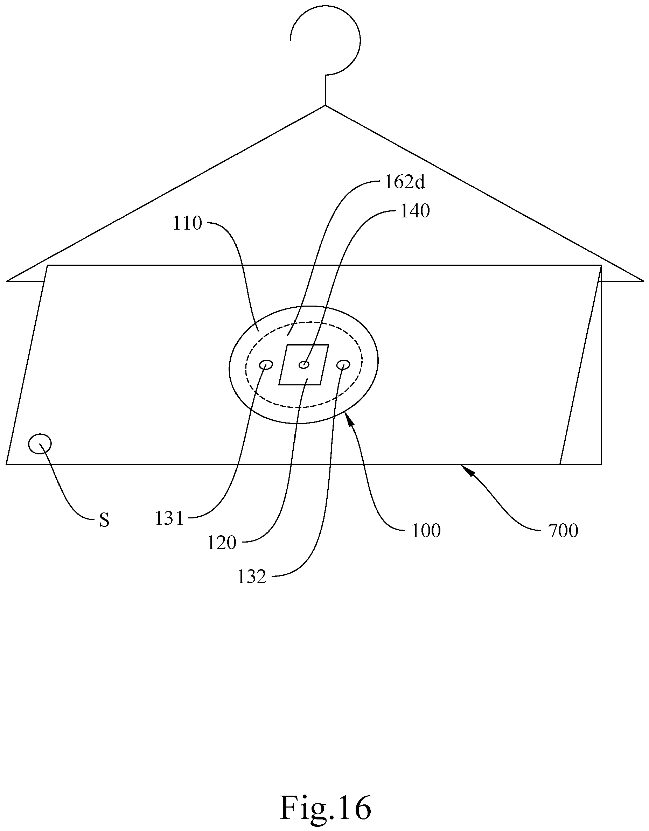

In another aspect, FIG. 15A, FIG. 15B and FIG. 16 correspond to a double-surface sterilization device 700 using the safe sterilization unit 101 in the second embodiment of the present invention, FIG. 15A and FIG. 15B show a schematic cross-sectional view of the double-surface sterilization device 700 during use, and FIG. 16 shows a schematic diagram of the double-surface sterilization device 700 in a usage status. Referring to FIGS. 7, 8, 15A, 15B and 16, the double-surface sterilization device 700 includes two or more safe sterilization units 101. For this embodiment, the double-surface sterilization device 700 includes two safe sterilization units 101 connected by a connecting portion 750; specifically, each of the safe sterilization units 101 is further provided with a bottom plate 710, and the two safe sterilization units 101 are respectively connected with the connecting portion 750 via one ends of the bottom plates 710. Thus, each of the safe sterilization units 101 is pivotable with the connecting portion 750 as a shaft. In addition, the double-surface sterilization device 700 can be expanded from the connecting portion 750 as the center according to requirements, and presents an angle A (as shown in FIG. 15B), where the angle may be 0-180°, preferably 0°, 30°, 60°, 90°, 120°, 150° or 180°; and thus a piece of clothing C can be hung above the double-surface sterilization device 700.

Further, the double-surface sterilization device 700 further includes a fixing component 740, an indicator lamp 720 and a circuit board 730. The fixing component 740 is disposed on the bottom plate 710 and configured to fix the clothing C. The fixing component 740 may be a conventional component such as a fastener or an adhesive component, which is not limited herein. The indicator lamp 720 is configured to remind a user of a usage status of the double-surface sterilization device 700; and specifically, the indicator lamp 720 can be in signal connection with the safety switch 130 to show the situation that the safety switch 130 regulates power supply of the light source portion 140, thereby reducing the probability of affecting the user by escaped light. The circuit board 730 is a carrier for laying electronic components required by the double-surface sterilization device 700, preferably a printed circuit board (PCB), can also serve as the power supply portion 612 as described in the above embodiment, and is configured to be connected with various apparatuses of the double-surface sterilization device 700 and to regulate power input to the double-surface sterilization device 700.

As shown in FIG. 16, the double-surface sterilization device 700 can be hung on a conventional clothes hanger; and thus, a piece of clothing can be irradiated by light from the light source portion 140 in the lens portion 162d while being hung on the clothes hanger, so as to further achieve sterilization. In addition, the double-surface sterilization device 700 may further include a switch assembly S, which can be configured to enable the user to manually turn on/off the double-surface sterilization device; and the switch assembly S may be in different forms, but not limited to, touch control, voice control, etc.

In conclusion, compared with the prior art, the safe sterilization unit and the double-surface sterilization device including the same in the present invention enable the light source portion for emitting the ultraviolet rays to fully exert its sterilization function without causing accidental damage to a human body through a mechanism that the infrared emitting diode and the infrared receiving diode act as the safety switch.

The present invention has been described in detail above, but the above is only a preferred implementation pattern and embodiment of the present invention, and is not intended to limit the scope of the present invention. In other words, those of ordinary skill in the art change and modify the present invention in various ways to adapt to different uses and conditions without violating the spirit and scope of the present invention. Thus, the implementation pattern also belongs to the claims of the present invention.

### [Description of reference numbers]

- 100: safe sterilization unit
- 110: bearing part
- 120: base plate
- 130: safety switch
- 131: infrared emitting diode
- 132: infrared receiving diode
- 140: light source portion
- 150: moving unit
- 161: light-transmitting portion
- 162a: lens portion
- 163a: conical structure
- 163b: arc structure
- 500: shell
- 510: base
- 170: bottom plate
- 520: extension portion
- 540a: support frame
- OP: opening
- PL: accommodating space
- IC: controller
- D: object to be sterilized
- 600: double-surface sterilization device
- 610: sterilization device body
- 611: outer shell
- S1: first side
- S2: second side
- PA1: first disinfection zone
- PA2: second disinfection zone
- SP1: first support frame
- SP2: second support frame
- CA1: first accommodating groove
- CA2: second accommodating groove
- 612: power supply portion
- CT: electrical connector
- A1: power supply module
- A2: charging module
- A3: battery
- 613: operation unit
- 614: guide groove
- SL: sliding mechanism
- SL1: sliding rail
- SL2: sliding block
- SL21: groove
- 615: positioning mechanism
- PT: positioning column
- SF: positioning elastic piece
- ST: accommodating mechanism
- 616: draw-out hook
- D1: distance
- DA1: disinfection range
- 700: double-surface sterilization device
- 710: bottom plate
- 720: indicator lamp
- 730: circuit board
- 740: fixing component
- 750: connecting portion
- A: angle
- S: switch assembly

## Claims

1. A safe sterilization unit (100), comprising:
a bearing portion (110) comprising two surfaces corresponding to each other;
a light source portion (140) configured to generate sterilization light, and disposed on one surface of the bearing portion (110); and
at least one safety switch (130) disposed on one side surface, provided with the light source portion (140), of the bearing portion (110), and connected with the light source portion (140),
wherein the safety switch (130) is disposed to sense light to generate a sensing message, and to regulate power supply of the light source portion (140) according to the sensing message, wherein
the light source portion (140) preferably comprises a light-emitting lamp body for emitting ultraviolet rays.

2. The safe sterilization unit according to claim 1, further comprising a lens portion (162a) disposed to contact the bearing portion (110) and cover the light source portion (140), and made from a vinyl aromatic and conjugated diene cyclic block copolymer.

3. The safe sterilization unit according to claim 1 or 2, further comprising a shell (500) formed with an opening (OP) communicating with the outside, wherein the bearing portion is disposed in the shell, and the light source portion is disposed on one surface, facing the opening, of the bearing portion.

4. The safe sterilization unit according to claim 3, wherein the safety switch (130) comprises an infrared emitting diode (131) and an infrared receiving diode (132); and the sterilization light and infrared rays emitted by the infrared emitting diode (131) both pass through the opening (OP), and the at least one safety switch (130) generates the sensing message according to an infrared ray receiving condition of the infrared receiving diode (132), wherein a light-transmitting portion (161) is preferably disposed at the opening (OP).

5. The safe sterilization unit according to claim 4, wherein the shell (500) comprises a lens portion (162a; 162b) disposed on the opening (OP), and covering the light source portion (140) and the safety switch (130); and a lens of the lens portion (162a; 162b) is a Fresnel lens.

6. The safe sterilization unit according to claim 4, wherein a lens portion (162c) is disposed on a lamp bead of the light source portion (140).

7. The safe sterilization unit according to any of the preceding claims, further comprising a moving unit (150) connected with the light source portion (140) to move an irradiation block of the light source portion.

8. A double-surface sterilization device (600), comprising:
a sterilization device body (610) comprising an outer shell (611), and a power supply portion (612) disposed inside the outer shell to regulate input power supply; and
two safe sterilization units (100) according to any of claims 1 to 7, the two safe sterilization units being electrically connected to the power supply portion (612) to obtain a driving power supply, and respectively disposed on two opposite sides of the outer shell (611) to form a first sterilization zone (PA1) on a first side of the outer shell and a second sterilization zone (PA2) on a second side of the outer shell.

9. The double-surface sterilization device according to claim 8, wherein a position, corresponding to the first disinfection zone (PA1), of the first side of the sterilization device body is provided with a first support frame (SP1); and
a position, corresponding to the second disinfection zone (PA2), of the second side of the sterilization device body is provided with a second support frame (SP2).

10. The double-surface sterilization device according to claim 9, wherein
the first support frame (SP1) is pivoted on the first side to adjust an opening and closing angle of the first support frame relative to a surface of the first side, and the second support frame (SP2) is pivoted on the second side to adjust an opening and closing angle of the second support frame relative to a surface of the second side; and/or wherein
the outer shell (612) has the first side formed with a first accommodating groove (CA1) for accommodating the first support frame (SP1), and the second side formed with a second accommodating groove (CA2) for accommodating the second support frame (SP2).

11. The double-surface sterilization device according to claim 8, wherein the sterilization device body (610) comprises two sliding mechanisms (SL) respectively disposed on the first side and the second side; and each of the two sliding mechanisms comprises sliding rails (SL1) disposed on the sterilization device body (610), and a sliding block (SL2) disposed on the sliding rails for bearing the corresponding safe sterilization unit.

12. The double-surface sterilization device according to claim 11, wherein the sterilization device body (610) comprises two guide grooves (614) respectively formed in the first side and the second side, respectively allowing shells of the safe sterilization units (100) on two sides to pass through, and limiting the movement stroke of the safe sterilization units (100), wherein
the sliding rails (SL) are preferably formed on two sides of each of the guide grooves (614) such that the outer shell protrudes toward an inner space, two sides of each of the sliding blocks are preferably formed with grooves for the sliding rails (SL) to be clamped, and the sliding block (SL2) is preferably limited to move linearly by the sliding rails (SL).

13. The double-surface sterilization device according to any of claims 8 to 12, wherein the sterilization device body comprises two positioning mechanisms (615) respectively disposed on the first side and the second side for fixing an object to be sterilized, wherein
each of the positioning mechanisms preferably comprises a positioning column (PT) inserted into the outer shell, and a positioning elastic piece (SF) with one end fixed to the positioning column; and the positioning elastic piece is preferably opened relative to the other end of the positioning column and elastically returns against a surface of the outer shell to provide clamping.

14. The double-surface sterilization device according to any of claims 8 to 13, wherein the power supply portion (612) is connected to an external power supply via an electrical connector disposed on the outer shell.

15. The double-surface sterilization device according to any of claims 8 to 14, wherein an inner side of the outer shell is provided with an accommodating mechanism (ST), the accommodating mechanism is open toward a bottom side of the outer shell, and a draw-out hook (616) is disposed in the accommodating mechanism.
